# EUROPEAN PATENT APPLICATION

(11) **EP 4 751 579 A1**
(43) Date of publication of application: **03.06.2026**
(21) Application number: 24216327.7
(22) Date of filing: 29.11.2024
(51) Int. Cl.: A23L 33/105, A61K 36/45, A61K 36/53, A61K 36/73, A61K 36/82, A61K 36/18, A61K 36/185, A61K 36/534, A61K 36/752, B01D 11/02

(54) **PLANT EXTRACTS WITH MULTICOMPONENT NATURAL DEEP EUTECTIC SOLVENT (NADES)**

(71) Applicant: NADES Design d.o.o., 10000 Zagreb (HR)
(72) Inventor: PANIC, Manuela, 10000 Zagreb (HR); RADOJCIC REDOVNIKOVIC, Ivana, 10000 Zagreb (HR)
(74) Representative: Hadzija, Tomislav

(57) **Abstract**

The invention relates to plant extract composition obtained by the use of multicomponent NADES wherein Choline bitartrate is used as one of the NADES components.

It provides a preparation method for extracting biocomponents from specific plants to obtain a ready-to-use food extracts.

The sensory properties of plant-based NADES extracts comprising choline bitartrate are superior to those comprising choline chloride.

## Description

### FIELD OF THE INVENTION

The present invention relates to multicomponent eutectic solvent and their use in extracting natural biological compounds. More particularly, invention relates to plant-based extracts with natural deep eutectic solvent (NADES) containing Choline bitartrate. Extracts are ready-to-use for the manufacture of food supplements without separation of NADES components.

### BACKGROUND OF THE INVENTION

Deep eutectic solvents (DES) have gained significan attention in recent years due to their unique properties and potential applications. These solvents are generally liquid at ambient temperature, whereas the compounds which the mixtures are composed of are solid compounds when considered separately. The phenomen of lowering melting points by the formation of a eutectic mixture is attributed to the establishment of inter-molecular hydrogen bonds, which have the effect of increasing the volume of the space between the chemical species thereby increasing their mobility such that they are rendered liquid.

Advantages compared to traditional solvens may be seen in their biodegradability and low toxicity. Rente at al. (Journal of Cleaner Production 380 (2022) 135147) gives a brief overview of the current knowledge regarding the use of DES/NADES as extraction solvents. Application of DES in the cosmetics industry and their relevance to green and sustainable chemistry are presented.

Natural deep eutectic solvents (NADES) relates to environmentally friendly biocompatible eutectic solvent compoused of naturally ocuring substances such as organic acid, sugars, amino acids etc and may be applied for the extraction of active natural components from plants. As a new class of green solvents with exceptional structural flexibility, it provides the opportunity for rational solvent design. Their extraction ability, viscosity at ambient temperature and other characteristics are to be fine-tuned with the selection of NADES components and their ratio.

A broad range of bioactive chemical compounds can be derived from plants. Plant extracts derived from various parts of plants are rich in antioxidants (like polyphenols), vitamins, and minerals, contributing to the health of human body. They represents an excellent source of molecules for the production of food additives, functional foods, nutritional products, and nutraceuticals.

Rosehip (*Rosae pseudo-fructus*): Rosehip contains high levels of vitamin C, polyphenols, and essential fatty acids. These compounds are known to provide antioxidant and anti-inflammatory benefits, supporting immune health, skin rejuvenation, and joint function. This ingredient is particularly suited for formulations targeting overall wellness and anti-aging properties.

Elderflower (*Sambuci flos*): Elderflower, rich in flavonoids and phenolic acids, demonstrates antioxidant and immune-enhancing effects. It has traditionally been utilized for respiratory support and the alleviation of cold symptoms, making it a suitable component in compositions aimed at immune health and cold symptom relief.

Cranberry (*Vaccinii macrocarpi fructus*): Cranberry contains proanthocyanidins, which inhibit bacterial adhesion within the urinary tract, supporting urinary health. Additionally, its high antioxidant content contributes to cardiovascular support, positioning cranberry as an ideal ingredient for heart health and infection-prevention formulations.

Green Tea (*Camelliae sinensis folium*): Green tea, rich in catechins such as epigallocatechin gallate (EGCG), possesses significant antioxidant, anti-inflammatory, and metabolism-boosting properties. These properties support cardiovascular health and metabolic regulation, enhancing green tea's applicability in weight management and heart health products.

Clubmoss (*Lycopodii herba*): Clubmoss contains bioactive alkaloids with potential neuroprotective and anti-inflammatory properties. Its neuroprotective qualities make it a valuable addition to formulations that target cognitive support and mental wellness.

Rosemary (*Rosmarini folium*): Rosemary contains rosmarinic acid and camosic acid, known for their preservative, antioxidant, and anti-inflammatory properties. Rosemary's preservative qualities and ability to support cognitive function and circulation enhance its use in functional food products with extended shelf life and cognitive benefits.

Chaste Tree (*Agni casti fructus*): Chaste tree fruit is known for its hormone-regulating properties, particularly in managing symptoms of premenstrual syndrome (PMS) and hormone balance. Its bioactive compounds, including agnuside, contribute to anti-inflammatory and mood-stabilizing effects, making it suitable for formulations addressing hormonal health.

Passion Flower (*Passiflorae herba*): Passion flower contains flavonoids with anxiolytic effects, known to interact with GABA receptors to promote relaxation. This ingredient is commonly used in calming formulations and can support mental relaxation, stress reduction, and sleep.

Nettle (*Urticae folium*): Nettle, rich in vitamins and minerals, has anti-inflammatory, diuretic, and detoxifying properties. It is suitable for use in formulations targeting joint health, detoxification, and immune support, thanks to its high mineral content and mild antihistamine effects.

Lady's Mantle (*Alchemilla vulgaris*): Lady's Mantle is rich in tannins, flavonoids, and phytoestrogens, which contribute to its astringent, antioxidant, and hormone-balancing properties. Traditionally used in herbal remedies, it is particularly valued for supporting women's health, including alleviating symptoms of menopause such as hot flashes and hormonal imbalances.

Hemp (*Cannabis sativa* L.): Hemp seeds are valued for their protein, fiber, and essential fatty acid content, beneficial for cardiovascular and digestive health. Cannabidiol (CBD) derived from hemp exhibits calming and anti-inflammatory effects, making it suitable for inclusion in food products aimed at wellness and mental relaxation.

Lime (*Citrus aurantiifolia*): Lime is high in vitamin C and flavonoids, providing antioxidant and immune-boosting effects. It is commonly used in beverages and other consumables for its antimicrobial, digestive-supportive, and refreshing qualities.

Spearmint (*Menta viridis*): Spearmint contains menthol and other volatile compounds that provide digestive and anti-inflammatory benefits. It is suitable for inclusion in foods and beverages targeting digestive health and mental clarity, providing a refreshing flavor and mild soothing effects.

Natural deep eutectic solvents (NADES) can be obtained by mixing two or more natural occurring compounds (alcohols, sugars, amino acids, natural acids, polyols, choline, etc.), wherein at least one of the NADES components acts as a hydrogen bond donor (HBD) and at least one NADES component acts as a hydrogen bond acceptor (HBA).

Components used for the preparation of NADES according to present invention may also enhance nutritional value of extracts obtained by contacting a plant material with said NADES which remain an integral part of final ready-to-use extract. Components used for the preparation of NADES according to present invention include Choline Bitartrate, Betaine, Sorbitol, Sucrose, Proline, Fructose, Glycerol and L-Carnitine.

Choline Bitartrate is a water-soluble compound formed by choline and tartaric acid, known for supporting brain function, memory, muscle control, and liver health through acetylcholine synthesis. In dietary supplements, it aids cognitive health and liver function.

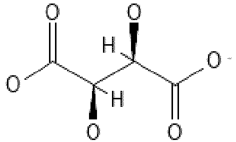

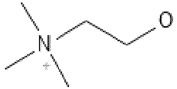

Betaine: A naturally occurring compound found in foods like beets, spinach, and grains. In the context of food, betaine is valued for its role in promoting liver health, supporting metabolic functions, and aiding digestion. It is commonly included in functional foods and dietary supplements for its potential to enhance athletic performance and improve cellular hydration.

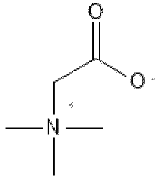

Sorbitol: A sugar alcohol occurring naturally in fruits, valued as a sugar substitute in food due to its low glycemic index. Known for its moisture-retaining properties, sorbitol is widely applied in personal care products, pharmaceuticals, and foods requiring hydration and smooth texture, like sugar-free and low-calorie foods.

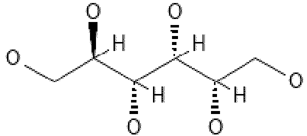

Sucrose: A disaccharide of glucose and fructose, commonly known as table sugar. It imparts sweetness and viscosity in formulations, enhancing flavor and texture. In food formulations, it helps manage osmotic properties, sweetness, and stability, working in conjunction with ingredients like betaine to balance moisture and texture.

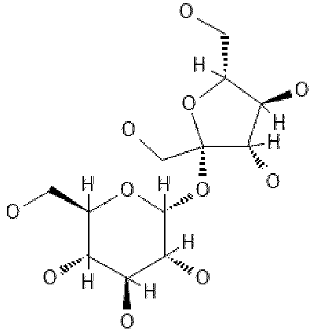

L-Carnitine: A natural compound involved in fat metabolism, L-camitine transports long-chain fatty acids into mitochondria for energy production. Frequently used in supplements for weight management and physical performance.

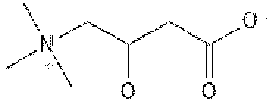

Proline: An amino acid naturally occurring in proteins, valued for its role in collagen synthesis and skin health. Proline supports tissue repair and hydration, making it a key ingredient in skincare formulations and supplements aimed at promoting elasticity and reducing signs of aging. Additionally, it is utilized in sports nutrition for its role in energy production and recovery.

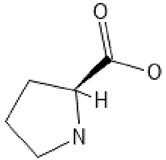

Fructose: A natural sugar found in fruits and honey, appreciated for its high sweetness and low glycemic index. Fructose is widely used as a sweetener in beverages and processed foods, particularly in products targeting diabetics or low-calorie formulations. Its humectant properties also make it useful in preserving moisture and enhancing texture in food products.

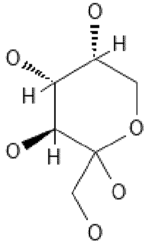

Glycerol: A natural polyol compound widely used in various industries for its humectant, emollient, and sweetening properties. Glycerol is valued for its ability to retain moisture, improve texture, and enhance the stability of formulations. Commonly found in personal care products, pharmaceuticals, and food items, it serves as a sweetener, solvent, and preservative. Its versatility makes it a key ingredient in moisturizing skincare products, syrups, and low-calorie foods.

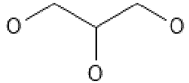

The present invention relates to a plant extracts obtained by extraction specific combination of plants, using NADES wherein choline bitartrate is used as hydrogen bond acceptor.

Several prior arts describe choline chloride as a component in NADES solvents dealing with extraction bioactive plant components (CN107383930, KR20210157614, KR102276980, CN114874173, US2023302073).

The present invention surprisingly found that plant extracts obtained by extraction with NADES comprising choline bitartrate has advantageous sensory properties over plant extracts obtained by extraction with NADES comprising choline chloride.

### SUMMARY OF THE INVENTION

The present invention relates to plant extracts obtained by contacting a plant material with a NADES (Natural Deep Eutectic Solvent), wherein the NADES comprises:
a. Choline bitartrate;
b. Two or three hydrogen bond donor selected from Sorbitol, Sucrose, Proline, Fructose, Glycerol and L-camitine; and
c. Water.

Plant materials are selected from:
i. Rosehip fruit
ii. Elderflower
iii. Cranberry
iv. Green tea leaf
v. Clubmoss herb
vi. Rosemary leaf
vii. Chaste tree fruit
viii. Passionflower herb
ix. Nettle leaf
x. Hemp flowers
xi. Lime
xii. Nana mint Moroccan
xiii. Lady's Mantle.

NADES solvent according to present invention may further comprises Betaine.

The present invention also relates to use of plant extracts for the manufacture of nutraceutical compositions.

### DETAILED DESCRIPTION OF THE INVENTION

Nutraceuticals as used herein relates to compositions containing bioactive compounds found in plants that may offer health benefits such as supporting immune health, reducing inflammation, promoting respiratory health, supporting urinary tract health, boosting metabolism and cardiovascular health, enhancing cognitive health, aiding digestion, maintaining hormonal balance, promoting relaxation, supporting joint health, relieving stress, and providing antioxidant, anti-inflammatory, and soothing properties.

Plant extract or NADES extract or plant-based NADES extract as used herein relates to extract obtained by extraction of bioactive components from plant using NADES wherein NADES remains an integral part of plant extract.

NADES as used herein relates to multicomponent liquid Natural Deep Eutectic Solvent obtained by mixing at least one hydrogen bond acceptor, two or three hydrogen bond donor and water.

NADES components according to present invention are edible and obtained extracts may be ready-to-use as food suplements without removing NADES. Furthermore, NADES enhances the efficacy of the final extract, resulting in a significantly improved effect in the end extract. As such, NADES extracts may be directly used in food industry. NADES extracts, due to their natural composition and inherent bioactivity, can be used directly as food supplements without the need for complex purification processes. Their ability to enhance the efficacy of extracted compounds results in significantly improved bioavailability and activity of the final product, offering benefits such as increased antioxidant capacity, enhanced stability, and better solubility.

By eliminating downstream purification, the production process becomes more cost-effective, energy-efficient, and environmentally friendly. Additionally, retaining the naturally occurring compounds that work together synergistically within NADES preserves the full range of bioactive properties, enhancing their overall health benefits. This streamlined approach reduces waste and aligns with sustainable production goals, making NADES extracts ideal for clean-label, ready-to-use applications in food.

NADES according to present invention are multicomponent, meaning that it consists of at least 3 components plus water. One of the advantages of multi-component NADES solvents is the ability to incorporate multiple bioactive components within the same solvent, enabling synergistic effects without exceeding the recommended daily doses specified for dietary supplements.

For instance, The European Food Safety Authority (EFSA) indicates that betaine can be included in dietary supplements with a maximum recommended dose of 1.5 g per day for adults, ensuring safe consumption levels. For sorbitol, as per Regulation (EU) No. 231/2012 and standard product formulations, it is commonly used in doses such as 4370 mg per 5 mL in syrups, remaining well within the safe range for its intended use.

This highlights the advantage of multi-component NADES solvents, allowing for precise inclusion of multiple bioactive compounds without exceeding daily limits, ensuring product safety and regulatory compliance in dietary supplements.

When used in food industry, sensory properties of obtained extracts are impotrant. The present invention disclose a combination of NADES components with good extraction ability and acceptable sensory properties at the same time.

In one embodiment of the present invention NADES comprises Choline bitartrate as hydrogen bond acceptor, wherein hydrogen bond donor are selected from Sorbitol, Sucrose, Proline, Fructose, Glycerol and L-camitine. In further embodiment NADES further comprises Betaine.

In one embodiment of the present invention NADES comprises Choline bitartrate as hydrogen bond acceptor; Sorbitol and Sucrose as hydrogen bond donor. In further embodiment NADES further comprises Betaine. In yet further embodiment NADES further comprises L-camitine.

In one embodiment of the present invention NADES consists of Choline bitartrate, Sorbitol, Sucrose and water.

In one embodiment of the present invention NADES consists of Choline bitartrate, Sorbitol, Sucrose, L-camitine and water.

In one embodiment of the present invention NADES consists of Choline bitartrate, Betaine, Sorbitol, Sucrose and water.

In one embodiment of the present invention NADES consists of Choline bitartrate, Betaine, Sorbitol, Sucrose, L-camitine and water.

In one embodiment NADES consists of Choline bitartrate, Proline, Sucrose and water.

In one embodiment NADES consists of Choline bitartrate, Betaine, Proline, Sucrose and water.

In one embodiment NADES consists of Choline bitartrate, Betaine, Proline, Sucrose, L-camitine and water.

In one embodiment NADES consists of Choline bitartrate, Fructose, Sucrose and water.

In one embodiment NADES consists of Choline bitartrate, Betaine, Fructose, Sucrose and water. In one embodiment NADES consists of Choline bitartrate, Betaine, Fructose, Sucrose, L-camitine and water.

In one embodiment NADES consists of Choline bitartrate, Glycerol, Sucrose and water.

In one embodiment NADES consists of Choline bitartrate, Betaine, Glycerol, Sucrose and water.

In one embodiment NADES consists of Choline bitartrate, Betaine, Glycerol, Sucrose, L-camitine and water.

In one embodiment NADES consists of Choline bitartrate, Fructose, Proline and water.

In one embodiment NADES consists of Choline bitartrate, Betaine, Fructose, Proline and water.

In one embodiment NADES consists of Choline bitartrate, Betaine, Fructose, Proline, L-camitine and water.

In one embodiment NADES consists of Choline bitartrate, Fructose, Sorbitol and water.

In one embodiment NADES consists of Choline bitartrate, Betaine, Fructose, Sorbitol and water.

In one embodiment NADES consists of Choline bitartrate, Betaine, Fructose, Sorbitol, L-camitine and water.

In one embodiment NADES consists of Choline bitartrate, Fructose, Glycerol and water.

In one embodiment NADES consists of Choline bitartrate, Betaine, Fructose, Glycerol and water.

In one embodiment NADES consists of Choline bitartrate, Betaine, Fructose, Glycerol, L-camitine and water.

In one embodiment NADES consists of Choline bitartrate, Proline, Sorbitol, and water.

In one embodiment NADES consists of Choline bitartrate, Betaine, Proline, Sorbitol and water.

In one embodiment NADES consists of Choline bitartrate, Betaine, Proline, Sorbitol, L-camitine and water.

In one embodiment NADES consists of Choline bitartrate, Betaine, Fructose, Proline, Sorbitol, and water.

In one embodiment NADES consists of Choline bitartrate, Betaine, Fructose, Proline, Glycerol, and water.

In one embodiment NADES consists of Choline bitartrate, Betaine, Fructose, Sorbitol, Glycerol, and water.

In one embodiment NADES consists of Choline bitartrate, Betaine, Proline, Sorbitol, Glycerol, and water.

In one embodiment of the present invention proportion of water in NADES is 20 to 50% by weight.

In further embodiment proportion of water in NADES is 25-35% by weight. In jet further embodiment proportion of water in NADES is 30 % by weight.

In one embodiment of the present invention NADES consist of Choline bitartrate, Sorbitol and Sucrose in molar ratio 1-4:1-4:1-4 with addition of 30% by weight of water.

In one embodiment of the present invention NADES consist of Choline bitartrate, Sorbitol, Betaine and Sucrose in molar ratio 1-4:1-4:1-4:1-4 with addition of 30% by weight of water.

In further embodiment of the present invention NADES consist of Choline bitartrate, Sorbitol, Betaine and Sucrose in molar ratio 1:1:1:1 with addition of 30% by weight of water.

In one embodiment of the present invention NADES consist of Choline bitartrate, Sorbitol, Betaine, Sucrose and L-camitine in molar ratio 1-4:1-4:1-4:1-4:0,5-2 with addition of 30% by weight of water.

In further embodiment of the present invention NADES consist of Choline bitartrate, Sorbitol, Betaine, Sucrose and L-camitine in molar ratio 1:1:1:1:0,5 with addition of 30% by weight of water.

In one embodiment of the present invention molar ratio between any NADES component in the mixture is 1:1-1:4.

In one embodiment of the present invention molar ratio between any NADES component in the mixture is 1:1.

In another embodiment, the NADES composition may further comprises food-grade components selected from: mannitol, trehalose, inositol, erythritol, glucose, maltose, mannose, rhamnose, xylose, glycine, alanine, valine, serine, isoleucine, histidine, glutamine, aspartic acid and urocanic acid.

In one embodiment of the present invention plant extracts are obtained by contacting a plant material with a NADES wherein plant material is selected from of Rosehip fruit, Elderflower and Cranberry.

In another embodiment of the present invention, plant extracts are obtained by contacting a plant material with a NADES, wherein the plant material is selected from Green tea leaf, Rosemary leaf, and Nettle leaf.

In another embodiment, plant extracts are obtained by contacting a plant material with a NADES, wherein the plant material is selected from Rosehip fruit, Elderflower, and Hemp flowers.

In one embodiment of the present invention plant extracts are obtained by contacting a plant material with NADES, wherein plant material is selected from Green tea leaf, Clubmoss herb and Rosemary leaf.

In another embodiment, plant extracts are obtained by contacting a plant material with a NADES, wherein the plant material is selected from Cranberry, Green tea leaf, and Lime.

In another embodiment, plant extracts are obtained by contacting a plant material with a NADES, wherein the plant material is selected from Rosemary leaf, Nana mint (Moroccan), and Elderflower.

In another embodiment, plant extracts are obtained by contacting a plant material with a NADES, wherein the plant material is selected from Green tea leaf, Clubmoss herb, and Rosemary leaf.

In one embodiment of the present invention plant extracts are obtained by contacting a plant material with NADES, wherein plant material is selected from Chaste tree fruit, Passionflower herb and Nettle leaf.

In another embodiment, plant extracts are obtained by contacting a plant material with a NADES, wherein the plant material is selected from Rosemary leaf, Hemp flowers, and Lime.

In another embodiment, plant extracts are obtained by contacting a plant material with a NADES, wherein the plant material is selected from Nettle leaf, Lime, and Rosemary leaf.

In another embodiment, plant extracts are obtained by contacting a plant material with a NADES, wherein the plant material is selected from Passionflower herb, Chaste tree fruit, and Hemp flowers.

In another embodiment, plant extracts are obtained by contacting a plant material with a NADES, wherein the plant material is selected from Lime, Passionflower herb, and Rosemary leaf.

In another embodiment, plant extracts are obtained by contacting a plant material with NADES, wherein the plant material is selected from Lady's Mantle, Passionflower herb, and Hemp flowers.

In another embodiment, plant extracts are obtained by contacting a plant material with NADES, wherein the plant material is selected from Lady's Mantle, Rosemary leaf, and Nettle leaf.

In another embodiment, plant extracts are obtained by contacting a plant material with NADES, wherein the plant material is selected from Lady's Mantle, Lime, and Moroccan Mint.

In another embodiment, plant extracts are obtained by contacting a plant material with NADES, wherein the plant material is selected from Lady's Mantle, Chaste tree fruit, and Passionflower herb.

In one embodiment of the present invention plant extracts are obtained by contacting a plant material with NADES, wherein plant material is selected from Hemp flowers, Lime and Nana mint moroccan.

In another embodiment, plant extracts are obtained by contacting a plant material with a NADES, wherein the plant material is selected from Passionflower herb, Rosemary leaf, and Nettle leaf.

In another embodiment, plant extracts are obtained by contacting a plant material with a NADES, wherein the plant material is selected from Green tea leaf, Elderflower, and Lime.

In another embodiment, plant extracts are obtained by contacting a plant material with a NADES, wherein the plant material is selected from Nana mint (Moroccan), Hemp flowers, and Green tea leaf.

In another embodiment, plant extracts are obtained by contacting a plant material with a NADES, wherein the plant material is selected from Elderflower, Rosemary leaf, and Passionflower herb.

Preparation of liquid plant extract includes:
a. preparation of natural deep eutectic solvent (NADES) by homogenization of previously weighted components in a blender for 10-20 minutes at room temperature, followed by dilution of said mixture with 20-40% w/w of demineralized water, and heating of thus prepared homogeneous mixture at 50-70 °C for 15-60 minutes until the formation of a clear viscous liquid, and cooling of thus prepared NADES to room temperature
b. extraction of dried plant material by said NADES at 20-75 °C for 0.5-3 h,
c. separation of the resulting liquid extract by filtration and/or centrifugation, yielding a filtrate that represents ready-to-use extract mixture of bioactive components in NADES.

In one embodiment of the present invention plant extracts are obtained by contacting a plant material with a NADES at temperature range of 20-60 °C.

In further embodiment of the present invention plant extracts are obtained by contacting a plant material with a NADES at temperature range of 40-60 °C.

In further embodiment of the present invention plant extracts are obtained by contacting a plant material with a NADES at temperature of 50 °C.

The natural biological compounds that may be extracted from plant material by the method of the invention may include phenolic acids and esters, flavonoids, phenolic alcohols, stilbenes, carotenoids, alkaloids, lipids, phenylpropanoids, flavorings and other compounds known to have beneficial effects to the human body.

As NADES solvent used for extraction are edible and non-toxic, the obtained extract can be directly formulated in foodstuffs and beverages.

In one embodiment plant-based NADES extracts according to present invention have potential application as food additive with antioxidant property. The antioxidant properties particularly relates to NADES extracts obtained by extraction of Rosehip fruit, Elderflower, Cranberry, Green tea leaf, Rosemary leaf, Nettle leaf, Hemp flowers, Lime and Nana mint (Moroccan).

In one embodiment NADES extracts according to present invention have potential application as food additive with beneficial effect to memory. The neuroprotective and cognitive-enhancing properties particularly relate to NADES extracts obtained by extraction of Green tea leaf, Clubmoss herb, Rosemary leaf, Chaste tree fruit, Passionflower herb and Hemp flowers.

In one embodiment NADES extracts according to present invention have potential application as food additive with beneficial effect to women's health. The hormone-balancing, stress-reducing, and menopause-alleviating properties particularly relate to NADES extracts obtained by extraction of Chaste tree fruit, Lady's Mantle, Passionflower herb, Nettle leaf, Rosemary leaf, Hemp flowers and Lime.

In one embodiment NADES extracts according to present invention have potential application as food additive with beneficial effect to general wellbeing. The calming, anti-inflammatory, stress-relieving, mood-enhancing, rejuvenating, and overall wellness-promoting properties particularly relate to NADES extracts obtained by extraction of Hemp flowers, Lime, Nana mint Moroccan, Passionflower herb, Rosemary leaf, Nettle leaf, Green tea leaf and Elderflower.

In one embodiment NADES extracts according to present invention are ready to use goodness.

Different additional components like vitamins, minerals, antioxidants, amino acids, essential fatty acids, plant extracts, probiotics, prebiotics, flavonoids, enzymes, aromatic compounds, dietary fibers, adaptogens, peptides and pigments may be added to the NADES extract to obtain final goodness compositions having immune-boosting, energy-enhancing, stress-relief, anti-inflammatory, cognitive-enhancing, skin-health, digestive-support, cardiovascular-health, anti-aging, bone-strengthening, hormonal-balance, and /or detoxifying properties.

The remaining filtered plant material with residual NADES can be applied to a wide range of food products and innovative applications, including functional beverages, natural flavoring agents, health-boosting syrups, concentrated nutrient-rich bases for smoothies or sauces, gummy supplements, raw energy bars, nutritionally enriched bakery products, plant-based spreads, snack coatings, protein-enriched snacks, fermentation starters, natural thickeners, salad dressings, soup bases, ice cream inclusions, sustainable edible packaging materials, edible threads for meal plating, biodegradable edible wraps for dish presentation, and natural edible garnishes or coatings for culinary tools and containers.

### EXAMPLES

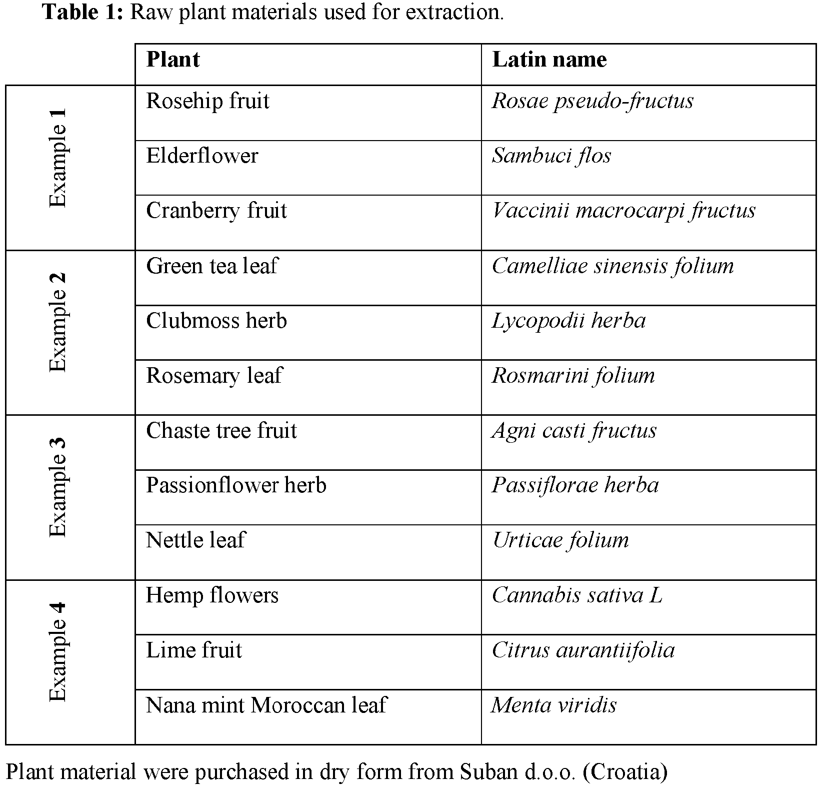

Following NADES mixtures were prepared for the extraction of phytochemicals from plant material:
NADES mixture 1:
   Choline bitartarate:sorbitol:betaine:sucrose in molar ratio 1:1:1:1 + water (30% by weight)
NADES mixture **1a**:
   Choline Chloride: sorbitol: betaine: sucrose in molar ratio 1:1:1:1 + water (30% by weight)
NADES mixture 2:
   Choline bitartarate:sorbitol:betaine:sucrose:L-camitine in molar ratio 1:1:1:1:0,5 + water (30% by weight)
NADES mixture **2a:**
   Choline chloride:sorbitol:betaine:sucrose:L-camitine in molar ratio 1:1:1:1:0,5 + water (30% by weight)

### Preparation of NADES:

The pre-weighed ingredients (HBA and HBD) were placed into a round-bottom flask, followed by the gradual addition of water. The preparation of NADES mixtures was conducted at 50 °C with gentle stirring for 60 minutes to 2 hours, or until a homogeneous, transparent, and colorless liquid was formed yielding NADES as an odorless and colorless viscous liquid.

### Solid Liquid Extractions - general procedure

For the extraction of phytochemicals, 1 g of raw plant materials was used per 10 mL of prepared NaDES. Extraction was carried out in a glass bottle at a constant temperature of 50°C and stirred for 2 hours. Extracts were then vacuum filtrated, separated and stored at +4 for further analysis. All extractions were made in triplicates for each mixture as well as for each plant.

### Example 1:

1g of Rosehip fruit, Elderflower and Cranberry (0,33 g of each plant) was extracted with 10 ml of NADES mixture **1** according to general procedure described above. After filtration, **Extract 1** was obtained in a viscous liquid form with an orange color.

### Comparative Example 1a:

1g of Rosehip fruit, Elderflower and Cranberry (0,33 g of each plant) was extracted with 10 ml of NADES mixture **1a** according to general procedure described above. After filtration, **Extract 1a** was obtained in a viscous liquid form with an orange color.

### Example 2:

1g of Green tea leaf, Clubmoss herb and Rosemary leaf (0,33 g of each plant) was extracted with 10 ml of NADES mixture **2** according to general procedure described above. After filtration, **Extract 2** was obtained in a viscous liquid form with a golden color..

### Comparative Example 2a:

1g of Green tea leaf, Clubmoss herb and Rosemary leaf (0,33 g of each plant) was extracted with 10 ml of NADES mixture **2a** according to general procedure described above. After filtration, **Extract 2a** was obtained in a viscous liquid form with a golden color..

### Example 3:

1g of Chaste tree fruit, Passionflower herb and Nettle leaf (0,33 g of each plant) was extracted with 10 ml of NADES mixture **1** according to general procedure described above. After filtration, **Extract 3** was obtained in a viscous liquid form with a brown color.

### Comparative Example 3a:

1g of Chaste tree fruit, Passionflower herb and Nettle leaf (0,33 g of each plant) was extracted with 10 ml of NADES mixture **1a** according to general procedure described above. After filtration, **Extract 3a** was obtained in a viscous liquid form with a golden color.

### Example 4:

1g of Hemp flowers, Lime and Nana mint Moroccan (0,33 g of each plant) was extracted with 10 ml of NADES mixture **2** according to general procedure described above. After filtration, **Extract 4** was obtained in a viscous liquid form with a golden-greenish color.

### The sensory testing were performed by following protocol:

The methodology of sensory testing was similar to those described in literature reference: Anand V, Kharb V, Kataria M, Kukka V, Choudhury PK. Taste assessment trials for sensory analysis of oral pharmaceutical products. Pak J Pharm Sci. 2008 Oct;21(4):438-50.

The testing was performed by six membered panel evaluated. Before the actual tasting, panels rinsed their mouths thoroughly with deionized water several times to neutralize any residual tastes. A small quantity of the extracts solution then administered. The extract was dropped on the spoon and placed directly on the tongue. The panel evaluated four main groups of sensory characteristics: taste (1), aftertaste (2), flavor (3), and texture/mouthfeel (4) and overall acceptability (5).

In the taste category, specific parameters such as sweetness (SW), saltiness (S), sourness (A), and bitterness (B) were assessed. Results are presented in Table 2.

Aftertaste was also evaluated, with particular attention to qualities such as bitterness (a), sweetness (b), saltiness (c), sourness (d), umami (e), astringency (f), numbness (g), and freshness (h). Results are presented in Table 3.

In the flavor group, assessments were made based on aroma, smell, and odor (S) as perceived through the nose; taste (T) as perceived in the mouth; and the overall balance of aroma and taste (F). Results are presented in Table 4.

Finally, the texture and mouthfeel group included evaluations of thinness (Tn), thickness (Tk), viscosity (Vs), and grittiness (Gy). Results are presented in Table 5.

The panel also evaluated overall acceptability. Results are presented in Table 6.

The participants provided an overall acceptability score for the product, typically using a 1-9 scale, where 1 represents extreme dislike and 9 represents extreme liking. Category scales such as 1= very weak, 5= medium, 9= very strong

**Table 2. The sensory tasting of extracts 1-3 and 4 versus extracts 1a-3a.**

| Extract No. | Taste intensity | | | | pH |
|---|---|---|---|---|---|
| | SW | S | A | B | |
| 1 | 7,8±1,3 | 8,6±0,9 | 6,8±1,3 | 8,2±1,1 | 4,1±0,0 |
| 2 | 6,3±2,3 | 7,8±1,5 | 4,8±1,8 | 6,7±2,0 | 4,6±0,0 |
| 3 | 6,3±2,3 | 7,0±2,8 | 5,8±2,1 | 6,7±2,6 | 4,2±0,0 |
| 4 | 8,0±1,3 | 9,0±0,0 | 8,0±0,9 | 8,0±1,1 | 4,7±0,0 |
| 1a | 6,2±2,3 | 3,6±2,1 | 6,2±0,8 | 2,2±1,1 | 4,8±0,0 |
| 2a | 4,7±2,7 | 4,8±1,5 | 6,5±2,3 | 3,8±2,3 | 5,5±0,0 |
| 3a | 6,4±2,7 | 3,6±1,5 | 4,8±2,7 | 2,8±1,6 | 6,2±0,0 |

| | | | | | |
|---|---|---|---|---|---|
| *sweetness SW, saltiness S, sourness A, and bitterness B | | | | | |

**Table 3. The sensory aftertaste of extracts 1-3 and 4 versus extracts 1a-3a.**

| | Aftertaste | | | | | | | |
|---|---|---|---|---|---|---|---|---|
| Extract No. | a | b | c | d | e | f | g | h |
| 1 | 8,2±0,8 | 8,2±0,8 | 8,8±0,4 | 7,8±0,4 | 8,4±0,5 | 7,8±1,3 | 8,8±0,4 | 8,8±0,4 |
| 2 | 6,5±1,2 | 6,3±2,4 | 7,8±1,5 | 6,2±2,3 | 6,8±2,0 | 6,3±2,2 | 7,8±2,9 | 7,2±1,5 |
| 3 | 6,7±2,2 | 6,7±2,6 | 7,2±2,5 | 6,5±2,3 | 7,0±2,4 | 6,7±2,3 | 7,8±2,0 | 7,0±2,4 |
| 4 | 8,2±0,7 | 8,6±0,5 | 8,2±0,7 | 8,2±0,7 | 8,2±0,7 | 7,8±1,2 | 8,8±0,4 | 8,4±0,8 |
| 1a | 2,4±1,1 | 6,0±1,7 | 3,4±1,1 | 5,2±1,8 | 4,0±1,0 | 4,6±2,6 | 7,8±2,7 | 3,6 ±1,1 |
| 2a | 4,0±2,1 | 4,7±1,6 | 4,3±1,5 | 5,3±2,6 | 4,7±1,6 | 4,8±2,5 | 8,2±2,0 | 4,3±1,9 |
| 3a | 3,0±1,4 | 5,2±3,1 | 3,4±1,7 | 4,8±2,6 | 3,6±1,5 | 4,6±2,9 | 7,8±2,7 | 3,2±1,3 |

| | | | | | | | | |
|---|---|---|---|---|---|---|---|---|
| *a - bitterness, b - sweetness, c - saltiness, d - sourness, e - umami, f - astringency, g - numbness, h - freshness | | | | | | | | |

**Table 4. The sensory flavor of extracts 1-3 and 4 versus extracts 1a-3a.**

| Extract No. | Flavour | | |
|---|---|---|---|
| | S | T | F |
| 1 | 8,0±1,2 | 7,8±0,4 | 7,6±1,1 |
| 2 | 6,7±1,9 | 5,5±1,2 | 6,0±1,3 |
| 3 | 7,7±1,0 | 6,2±2,6 | 6,5±2,0 |
| 4 | 7,4±1,0 | 7,6±1,2 | 7,4±1,4 |
| 1a | 6,0±2,5 | 3,0 ±1,6 | 3,0±1,2 |
| 2a | 7,0±1,4 | 3,3±2,2 | 3,0±0,6 |
| 3a | 5,4±2,2 | 3,0±2,3 | 2,8±1,9 |

| | | | |
|---|---|---|---|
| *S - aroma, smell, odor, T - taste, F - overall balance of aroma and taste | | | |

**Table 5. The sensory texture/mouthfeel of extracts 1-3 and 4 versus extracts 1a-3a.**

| Extract No. | Texture/mouthfeel | | | |
|---|---|---|---|---|
| | Tn | Tk | Vs | Gy |
| 1 | 8,4±0,9 | 8,4±0,9 | 8,8±0,4 | 8,8±0,4 |
| 2 | 7,5±1,2 | 7,2±1,2 | 7,5±1,0 | 8,5±0,8 |
| 3 | 8,0±0,6 | 8,2±0,8 | 8,3±0,8 | 7,5±2,3 |
| 4 | 8,2±0,7 | 8,2±0,7 | 8,2±0,7 | 6,2±1,3 |
| 1a | 7,0±2,1 | 6,6±2,5 | 7,0±2,1 | 8,4±0,9 |
| 2a | 6,0±2,2 | 6,8±1,7 | 6,8±1,7 | 8,0±1,7 |
| 3a | 6,0±2,8 | 6,4±2,4 | 6,8±1,8 | 8,2±0,8 |

| | | | | |
|---|---|---|---|---|
| *Tn - thinness, Tk - thickness, Vs - viscosity, Gy - grittiness | | | | |

**Table 6. Overall sensory acceptability**

| Extract No. | Overall acceptability |
|---|---|
| 1 | 7,8±0,4 |
| 2 | 6,3±0,8 |
| 3 | 7±1,3 |
| 4 | 7,8±0,8 |
| 1a | 3,6±1,1 |
| 2a | 4,3±1,8 |
| 3a | 3±1,7 |

Sensory results of **Extracts 1-3** compared with the results of **Extracts 1a-3a** show that the sensory properties of extracts obtained by NADES 1 and 2 comprising choline bitartrate is favorable over extracts obtained by NADES 1a and 2a comprising choline chloride.

The obtained extracts were also tested for their antioxidative activity according different standard methods, including:
• the Total Phenolic Content (TPC) assay (V. L. Singleton, R. Orthofer, R. M. Lamuela-Raventós: Analysis of Total Phenols and Other Oxidation Substrates and Antioxidants by Means of Folin-Ciocalteu reagent, Meth. Enzymol. 299 (1999) 152-178)
• the Oxygen Radical Absorbance Capacity (ORAC) assay (P. Ninfali, G. Mea, S. Giorgini, M. Rocchi, M. Bacchiocca:Antioxidant capacity of vegetables, spices and dressings relevant to nutrition, Brit. J. Nutr. 93 (2005) 257-266)

### Determination of Total Phenolic Content

Total phenolic content (TPC) was determined by the modified Folin-Ciocalteu method published by Panić et al. 2021 (Panić, M.; Gunjević, V.; Radošević, K.; Cvjetko Bubalo, M.; Ganić, K.K.; Redovniković, I.R.: COSMOtherm as an Effective Tool for Selection of Deep Eutectic Solvents Based Ready-To-Use Extracts from Graševina Grape Pomace, Molecules, Volume 26, 2021, Article 4722.). The reaction mixture consisted of 0.125 mL of 125 times diluted sample and 0.625 mL of 10 times Folin-Ciocalteu reagent. After 5 minutes of incubation at room temperature, 0.5 mL of Na2CO3 (75 g L⁻¹) was added and reaction was incubated 5 minutes in a hot water bath (50°C). After incubation, the absorbance was measured at 760 nm and results were expressed as mg of gallic acid equivalent per g of the used plant (mg GAE g⁻¹). Spectrophotometric analyses were conducted in triplicate. The results of total phenolic content (TPC) were presented in Table 7.

### Oxygen Radical Absorbance Capacity Assay (ORAC)

The antioxidant properties of extracts were measured by ORAC assay as described in Panić et al. 2019 (M. Panić, M. Radić Stojković, K. Kraljić, D. Skevin, I. Radojšić Redovniković, V. Gaurina Srček, K. Radošević: Ready-to-use green polyphenolic extracts from food by-products, *Food Chemistry,* Volume 283, 2019, Pages 628-636.)

Briefly, the antioxidant properties of extracts were measured by ORAC assay. The results were expressed as relative ORAC values. The assay was conducted in a quartz stone with 3 mL of reaction mixture: 2.25 mL of 0.04 µmol L⁻¹ fluorescein sodium salt in 0.075 M sodium phosphate buffer (pH 7.0), and 0.375 mL of 1000-10000 times diluted extracts or 25 mM Trolox as standard or 0.075 M sodium phosphate buffer (pH 7) as blank control. The reaction mixtures were incubated for 30 min at 37°C followed by reaction initiation with 0.375 mL 152 mm AAPH and fluorescence was read every minute up to value zero at 485 nm excitation, 520 nm emission by a Varian Cary Eclipse Spectrofluorimeter (Palo Alto, CA, USA). Results were analysed using the differences of areas under fluorescein decay curve between the blank and the sample. The results were the mean values (n=3) and were expressed as mmol Trolox equivalent per g of NADES (mmol TE g⁻¹). The results were presented in table 7.

**Table 7. Polyphenolic content (TP, mg gdw⁻¹ of plant material) and ORAC values (mmol TE g_{dw}⁻¹) of prepared extract. Results were expressed as the means (n=3) ± S.D.**

| Extract No. | | |
|---|---|---|
| | TPC | ORAC |
| 1 | 8,63±2,34 | 1404,89±67 |
| 1a | 9,94±0,3 | 1075,17±74 |
| 2 | 9,29±3,04 | 2522, 10±43 |
| 2a | 11,11±1,42 | 1477,18±43 |
| 3 | 6,36±0,56 | 1295,85±16 |
| 3a | 5,24±1,24 | 748,35±72 |
| 4 | 10,04±2,51 | 682±44 |

### Estimation of the sensory acceptability of NADES-based extracts

Electronic tongue analysis was used for the estimation of the sensory acceptability of NADES-based extracts.

Electronic tongue system (Astree 2, Alpha M.O.S.) consisted of seven potentiometric sensors for the application in food analysis, reference Ag/AgCl electrode, autosampler, and electronic unit connected to the computer was used according to the protocol described in Panić et al. (M. Panić, S. Drakula, G. Cravotto, R. Verpoorte, M. Hruškar, I. Radojčić Redovniković, K. Radošević: Biological activity and sensory evaluation of cocoa by-products NADES extracts used in food fortification, *Innovative Food Science* & *Emerging Technologies,* Volume 66, 2020, 102514.

## Claims

1. A plant extract obtained by contacting a plant material with a NADES, wherein said NADES comprises:
a. Choline bitartrate;
b. Two or three hydrogen bond donor selected from Sorbitol, Sucrose, Proline, Fructose, Glycerol and L-camitine; and
c. Water.

2. The plant extract according to claim 1, wherein plant material is selected from:
i. Rosehip fruit
ii. Elderflower
iii. Cranberry
iv. Green tea leaf
v. Clubmoss herb
vi. Rosemary leaf
vii. Chaste tree fruit
viii. Passionflower herb
ix. Nettle leaf
x. Hemp flowers
xi. Lime
xii. Nana mint Moroccan
xiii. Lady's Mantle.

3. The plant extract according to any of claims 1 to 2 wherein NADES further comprises Betaine.

4. The plant extract according to any of claims 1 to 3, wherein the proportion of water in NADES is from 20 - 50% by weight.

5. The plant extracts according to any of claims 1 to 4, wherein the proportion of water in NADES is 30% by weight.

6. The plant extract according to any of claims 3 to 5, wherein NADES comprises Choline bitartrate, Betaine, Sorbitol and Sucrose at the molar ratio 1-4:1-4:1-4:1-4.

7. The plant extract according to claims 6, wherein the molar ratio of Choline bitartrate: Betaine: Sorbitol: Sucrose is 1:1:1:1.

8. The plant extract according to any of claims 3 to 5, wherein NADES comprises Choline bitartrate, Betaine, Sorbitol, Sucrose and L-camitine at the molar ratio 1-4:1-4:1-4:1-4:0,5-2.

9. The plant extract according to claims 8, wherein the molar ratio of Choline bitartrate: Betaine: Sorbitol: Sucrose: L-carnitine is 1:1:1:1:0.5.

10. The plant extract according to any of claims 1 to 9, wherein plant material is selected from Rosehip fruit, Elderflower and Cranberry.

11. The plant extract according to any of claims 1 to 9, wherein plant material is selected from Green tea leaf, Clubmoss herb and Rosemary leaf.

12. The plant extract according to any of claims 1 to 9, wherein plant material is selected from Chaste tree fruit, Passionflower herb and Nettle leaf.

13. The plant extract according to any of claims 1 to 9, wherein plant material is selected from Hemp flowers, Lime and Nana mint Moroccan.

14. The plant extract according to any of claims 1-13, wherein plant extract is ready to use for food supplements.

15. Use of plant extract according to any of preceding claims for the manufacture of nutraceutical compositions.
